(19) Europäisches Patentamt
European Patent Office
Office européen des brevets

(11) Veröffentlichungsnummer : **0 023 293**
**B1**

(12) # EUROPÄISCHE PATENTSCHRIFT

(45) Veröffentlichungstag der Patentschrift : **01.06.83**

(51) Int. Cl.[3] : **C 07 D319/12**

(21) Anmeldenummer : **80103955.3**

(22) Anmeldetag : **10.07.80**

(54) **Verfahren zur Herstellung von 2.3-Perfluor- bzw. -trifluormethyl-1,4-dioxanen.**

(30) Priorität : **14.07.79 DE 2928602**

(43) Veröffentlichungstag der Anmeldung : **04.02.81 Patentblatt 81/05**

(45) Bekanntmachung des Hinweises auf die Patenterteilung : **01.06.83 Patentblatt 83/22**

(84) Benannte Vertragsstaaten : **BE DE FR GB IT NL**

(56) Entgegenhaltungen :
**DE A 2 648 123**
**DE A 2 843 767**
**US A 3 578 684**
**Houben-Weyl, Bd V/3 (1962), S. 155-157**
**Gould, Mechanismus und Struktur der Org. Chemie, S. 307-308 (1962)**
**Klages, Lehrbuch der Org. Chemie, S. 308-309 (1957)**

(73) Patentinhaber : **HOECHST AKTIENGESELLSCHAFT**
**Postfach 80 03 20**
**D-6230 Frankfurt/Main 80 (DE)**

(72) Erfinder : **Muffler, Herbert, Dr.**
**Tucholskystrasse 14**
**D-6000 Frankfurt am Main 70 (DE)**
Erfinder : **Siegemund, Günter, Dr.**
**Frankfurter Strasse 21**
**D-6238 Hofheim am Taunus (DE)**
Erfinder : **Schwertfeger, Werner, Dr.**
**Haydnstrasse 17**
**D-6308 Butzbach (DE)**

**EP 0 023 293 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 2.3-Perfluor- bzw. -trifluormethyl-1,4-dioxanen

Das einfachste 2.3-Perfluor-1.4-dioxan ist 2.2.3.3.-Tetrafluor-1.4-dioxan ; es entsteht neben verschiedenen anderen Produkten bei der katalytischen Hydrierung von Perfluor-1.4-dioxan-2 [P. L. Coe et al. J. of Fluorine Chemistry *6,* 115 bis 128 (1975)]. Seine Isolierung aus dem Reaktionsgemisch erfolgt durch Destillation, wobei der bei der katalytischen Hydrierung ebenfalls gebildete Ethoxydifluoressigsäuremethylester mit überdestilliert, und Trennung der beiden Verbindungen durch Umsetzung mit Natronlauge (wobei nur der Ester verseift wird). Aus dem Verseifungsgemisch wird das 2.2.3.3.-Tetrafluor-1.4-dioxan dann durch Extraktion mit Ether und übliche Aufarbeitung des Etherextraktes in etwa 3 %iger Ausbeute erhalten.

Mit wesentlich höheren Ausbeuten (bis etwa 45 % d. Th.) soll sich nach US-A-3 578 684 eine rein formelmäßig dem 2.2.3.3.-Tetrafluor-1.4-dioxan ähnliche Verbindung, nämlich das Perfluor-β-oxa-δ-valerolacton, durch Umsetzung von Perfluoroxydiacetylhalogeniden mit wasserfreien Alkalifluoriden bei Temperaturen von etwa 20 bis 200 °C herstellen lassen. Perfluor-β-oxa-δ-valerolacton gehört jedoch als (cyclischer) Ester einer Stoffklasse an, die sich von (cyclischen) Ethern (wie den vorerwähnten Dioxanderivaten) grundsätzlich unterscheidet. Die US-A-3 578 684 kann daher für die Herstellung entsprechender (cyclischer) Ether keine Anregung geben.

Wegen des schwierigen Zugangs zu dem 2.2.3.3.-Tetrafluor-1.4-dioxan sowie der von P. L. Coe et al., a. a. O. mitgeteilten völlig unzureichenden Ausbeute bestand die Aufgabe, ein verbessertes Verfahren zur Herstellung dieser Verbindungen zu entwickeln, das möglichst auch auf die Herstellung noch anderer 2.3-Perfluor-1.4-dioxane anwendbar ist. Das zu entwickelnde verbesserte Verfahren brauchte sich allerdings nicht anwenden lassen auf die Herstellung des 2.2.3.3.-Tetrakis-trifluormethyl-1.4-dioxans, da dessen Herstellung in praktisch quantitativer Ausbeute aus Perfluorpinakol und 1.4-Dioxan bereits bekannt ist [A.F. Eleev et al. Izv. Akad. Nauk SSSR, Ser Khim. *1978,* 509].

Die gestellte Aufgabe konnte erfindungsgemäß durch Umsetzung bestimmter Carbonylverbindungen mit Alkalifluoriden und/oder mit Ammoniumfluorid gelöst werden.

Erfindungsgegenstand ist somit ein Verfahren zur Herstellung von 2.3-Perfluor- bzw. -trifluormethyl-1,4-dioxanen der allgemeinen Formel I

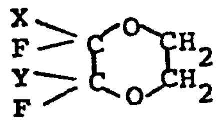

(I)

worin X und Y unabhängig voneinander F oder $CF_3$ bedeuten, das dadurch gekennzeichnet ist, daß man Carbonylverbindungen der allgemeinen Formel II

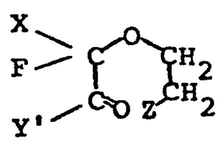

(II)

worin

X die gleiche Bedeutung wie in Formel I besitzt,

Y' die gleiche Bedeutung wie Y in Formel I hat (F, $CF_3$), und zusätzlich noch Cl sein kann, und

Z = Cl, Mesylat [= $OSO_2CH_3$]- oder Tosylat [= $OSO_2C_6H_4$—$CH_3$(p)]-rest,

mit Alkalifluoriden und/oder mit Ammoniumfluorid in einer Menge von mindestens etwa 1 Mol Fluorid/Mol Carbonylverbindung II, wenn Y' = F oder $CF_3$, oder von mindestens etwa 2 Mol Fluorid/Mol Carbonylverbindung II, wenn Y' = Cl, umsetzt.

Die Umsetzung läßt sich durch folgendes Reaktionsschema wiedergeben

a) wenn Y' = Y = F oder $CF_3$ :

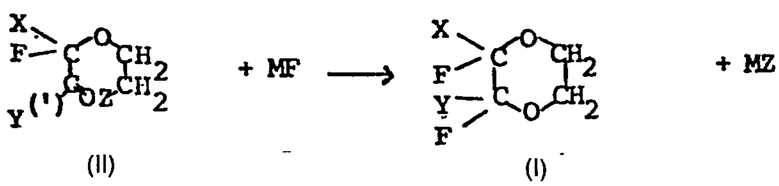


(II) (I)

b) wenn Y' = Cl :

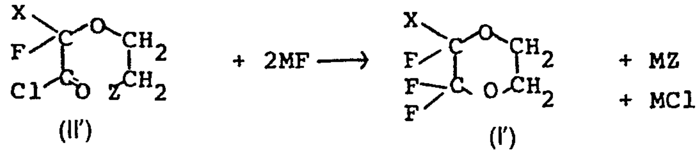


(II') (I')

M bedeutet jeweils ein Alkalimetall- oder Ammoniumion.
Formel II' = Formel II mit Y' = Cl,
Formel I' = Formel I mit Y = F.

Es war überraschend, daß bei dieser Umsetzung nicht — wie man zumindest zunächst hätte erwarten müssen — der Rest Z in den Ausgangs-Carbonylverbindungen gegen F ausgetauscht wird, sondern daß hier die entsprechenden 2,3-Perfluor- bzw. -trifluormethyl-1,4-dioxane entstehen.

Die Ausgangsverbindungen II für das erfindungsgemäße Verfahren sind teils bekannt (vgl. z. B. DE-OS 1 793 240) ; teils sind sie erhältlich ausgehend von den bekannten 2-Ethoxy-carbonsäureestern III :

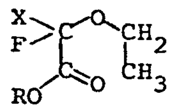

(III)

X besitzt hier die gleiche Bedeutung wie in Formel II und R bedeutet einen Alkylrest.

Die Ester III können etwa durch Verseifung mit NaOH in die Natriumsalze, und diese dann mit einem anorganischen Säurechlorid wie $POCl_3$ oder $SOCl_2$ in die Säurechloride überführt werden. Durch Umsetzung mit anorganischen Fluoriden wie z. B. mit KF entstehen aus den Säurechloriden die entsprechenden Säurefluoride. Deren Lichtchlorierung liefert dann die 2-(β-Chlorethoxy)-carbonsäurefluoride IV.

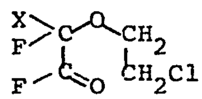

(IV)

Man erhält hier diejenigen Verbindungen II, in welchen Z = Cl ist.

Die Verbindungen II mit Z = Mesylat oder Tosylat werden verteilhaft in situ hergestellt durch Umsetzung der Fluorcarbonylverbindungen V

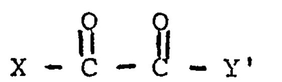

(V)

mit den Ethanderivaten VI

$$Z—CH_2—CH_2—Z \quad (VI),$$

wobei in den Formeln X und Y' die gleiche Bedeutung wie in Formel II besitzen und Z von den dortigen Bedeutungen nur = Mesyl oder Tosyl ist, und Alkalifluoriden und/oder mit Ammoniumfluorid.

Die Verbindungen V und VI werden zweckmäßig im Mol-Verhältnis von etwa 1 : 1 bis 1 : 1,5 eingesetzt. Wenn Y' = Y = F oder $CF_3$, beträgt die Menge des eingesetzten Fluorids mindestens etwa Mol pro Mol V oder VI. Wenn Y' = Cl, entsteht mit etwa 2 Mol Fluorid die Carbonylverbindung II (mit Y' = F). Mit weiterem Fluorid erfolgt dann der Ringschluß der Carbonylverbindungen II zu dem entsprechenden 2.3-Perfluor- bzw. -trifluormethyl-1,4-dioxan. Einer Isolation der Verbindungen II bedarf es hier nicht.

Die für das erfindungsgemäße Verfahren geeigneten Fluoride sind Alkalifluoride und/oder Ammoniumfluorid. Bevorzugte Fluoride sind KF und/oder CsF. Die Fluoride können einzeln oder im Gemisch eingesetzt werden. Zusatzstoffe wie z. B. HF oder sogenannte Kronenether (= sauerstoffhaltige Makro-Ringverbindungen) können mitverwendet werden.

Für das Gelingen der erfindungsgemäßen Umsetzung ist es im Prinzip nicht nötig, mehr als 1 oder 2 Mol Fluorid/Mol Carbonylverbindung II einzusetzen ; bevorzugt ist jedoch ein 5 bis 50 Mol-%iger Überschuß. Höhere Überschüsse bringen keinen Vorteil.

Weiterhin wird das Verfahren vorzugsweise in einem aprotischpolaren Lösungsmittel durchgeführt. Als solche Lösungsmittel kommen z. B. in Frage : Ether wie Diethylenglykoldimethylether oder Tetraethylenglykoldimethylether ; Nitrile wie Benzonitril ; Säureamide wie Dimethylformamid, Sulfoxide wie Dimethylsulfoxid, Sulfolan, etc. Die Reaktionstemperatur kann im allgemeinen zwischen 20 und 200 °C liegen ; vorzugsweise liegt sie zwischen 50 und 160 °C, insbesondere zwischen 60 und 130 °C.

Es kann sowohl bei Normaldruck als auch unter Überdruck gearbeitet werden.

Die Reaktionszeit beträgt im allgemeinen zwischen 5 und 35 Stunden.

Es ist für die erfindungsgemäße Reaktion praktisch ohne Belang, in welcher Reihenfolge die Reaktionskomponenten und das Lösungsmittel zusammengegeben werden. Es ist allerdings von Vorteil, durch gutes Rühren für eine gleichmäßige Durchmischung des Ansatzes zu sorgen :

Nach einer bevorzugten Arbeitsweise wird das Fluorid in einem aprotisch-polaren Lösungsmittel vorgelegt und mit der Ausgangs-Carbonylverbindung II versetzt. Anschließend wird das Gemisch erhitzt.

Das gebildete 2.3-Perfluor- bzw. -trifluormethyl-1,4-dioxan wird mittels einer Wasserdampfdestillation aus dem Reaktionsgemisch isoliert.

Wird die Ausgangs-Carbonylverbindung II ausgehend von einer Fluorcarbonylverbindung V und einem Ethanderivat VI hergestellt, so ist es vorteilhaft, das Fluorid in einem aprotisch-polaren Lösungsmittel vorzulegen, unter Eiskühlung die Fluorcarbonylverbindung V zuzugeben und das Gemisch anschließend mit dem Ethanderivat VI zu versetzen. Nach dem Erhitzen des Gemisches erfolgt die Isolierung des 2.3-Perfluor- bzw. -trifluormethyl-1,4-dioxans zweckmäßig wieder mittels einer Wasserdampfdestillation.

Die nach dem erfindungsgemäßen Verfahren hergestellten 2.3-Perfluor- bzw. -trifluormethyl-1,4-dioxane der Formel I sind farblose Flüssigkeiten, die sowohl gegen saure als auch basische Hydrolyse beständig sind. In organischen Lösungsmitteln sind sie gut löslich, mit Wasser dagegen nur wenig mischbar.

Das Verfahren gestattet die Herstellung von 2.3-Perfluor- bzw. -trifluormethyl-1,4-dioxane in relativ einfacher Weise mit Ausbeuten, die zwar noch nicht sehr gut, aber doch mindestens etwa um den Faktor 10 besser als nach dem bekannten Verfahren von Coe et al. a. a. O. sind die Ausbeuten betragen durchweg 10 bis 30 % d. Th., im Einzelfall ggf. auch bis zu etwa 50 %. Außerdem sind die 1.4-Dioxane der Formeln VII und VIII neue Verbindungen.

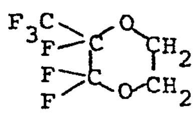

(VII)

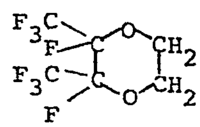

(VIII)

Die Verbindungen sind wertvolle Lösungsmittel für hochfluorierte Verbindungen sowie Zwischenprodukte auf verschiedenen Sachgebieten, z. B. auf dem Pharmagebiet.

Die Erfindung wird nun durch die nachfolgenden Beispiele näher erläutert.

Beispiel 1

In einem trockenen 500 ml Vierhalskolben mit Thermometer, Rückflußkühler, KPG-Rührer und Tropftrichter werden 83,6 g (0,55 Mol) CsF in 250 ml trockenem Diglyme vorgelegt. Zu dieser Mischung werden 77 g (0,44 Mol) $Cl—CH_2—CH_2—O—CF_2—COF$ in ca. 30 Min. zugetropft, wobei die Temperatur auf 44 °C ansteigt. Anschließend wird der Ansatz 8 h auf 110-120 °C erhitzt. Dabei wird die Mischung zunehmend dickflüssiger. Das erkaltete Reaktionsgemisch wird einer Wasserdampfdestillation unterworfen. Die dabei erhaltene schwerere Phase wird abgetrennt, über $Na_2SO_4$ getrocknet und über eine Füllkörperkolonne fraktioniert destilliert. Es werden 23 g (0,14 Mol) 2.2.3.3.-Tetrafluor-1.4-dioxan erhalten.

Siedepunkt : 119-121 °C/750 mm Hg (99991 Pa)

Ausbeute : 32,7 % d. Th.

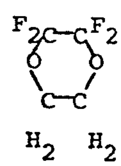

| | | | |
|---|---|---|---|
| MG : | 160 | S.F. : | $C_4H_4F_4O_2$ |
| Ber : | C 30,01 | H 2,52 | F 47,48 |
| Gef : | C 30,05 | H 2,50 | F 47,35 |

Beispiel 2

In einem 500 ml Rührkolben mit KPG-Rührer, Rückflußkühler, Thermometer und Tropftrichter, der unter Stickstoff ausgeheizt worden ist, werden 31 g (0,2 Mol) CsF in 200 ml trockenem Diglyme vorgelegt. 45 g (0,2 Mol) $Cl—CH_2—CH_2—O—CF(CF_3)—COF$ werden innerhalb von 90 Minuten bei 26-29 °C eingetropft. Anschließend wird das Reaktionsgemisch unter Rühren auf 120 °C erwärmt und ca. 10 Stunden lang bei dieser Temperatur gehalten. Mit einer Wasserdampfdestillation werden 35 g organisches Produkt aus dem Reaktionsgemisch abgetrennt. Sie enthalten 11 g (0,053 Mol) 2.3.3-Trifluor-2-trifluormethyl-1.4-dioxan.

Siedepunkt : 74,5-76,5 °C/100 mm Hg (13332 Pa)

Ausbeute : 26 % d. Th.

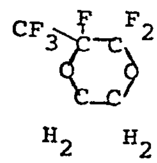

MG : 210 S.F. : $C_5H_4F_6O_2$
Ber : C 28,57 H 1,90 F 54,28
Gef : C 29,00 H 2,10 F 53,00

Beispiel 3

In einem ausgeheizten 1-Liter Rührkolben mit KPG-Rührer, Rückflußkühler, Thermometer und Gaseinleitungsrohr gibt man 64 g (1,1 Mol) KF und 500 ml trockenes Diglyme. In diese Mischung werden unter Eiskühlung 47 g (0,5 Mol) Oxalylfluorid eingeleitet. Danach wird das Gaseinleitungsrohr entfernt, und man gibt 109 g (0,5 Mol) Glykoldimesylat zu. Der Ansatz wird im Laufe von ca. 24 Stunden auf 85 °C aufgeheizt und 8 Stunden bei dieser Temperatur gehalten. Bei der nachfolgenden Wasserdampfdestillation werden 14 g organische Phase erhalten, die 8 g 2.2.3.3.-Tetrafluor-1.4-dioxan enthalten.

Ausbeute : 10 % d. Th.

Beispiel 4

Ansatz und Reaktionsdurchführung wie Beispiel 3.

Es werden jedoch noch 1,5 g (4,2 mMol) des sog. Kronenethers Dibenzo-18-Krone 6 zugesetzt ; die letztgenannte Verbindung ist 2.3.11.12-Dibenzo-1.4.7.10.13.16-hexaoxacycloocta-deca-2.11-dien und besitzt die Formel :

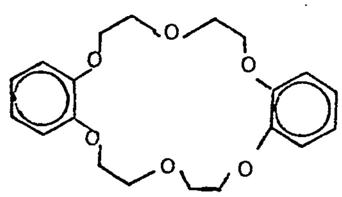

Ausbeute : 8,8 g 2.2.3.3.-Tetrafluor-1.4-dioxan ≙ 11 % d. Th.

Beispiel 5

Ansatz und Reaktionsdurchführung wie Beispiel 3. Es werden jedoch noch 7,5 g (0,05 Mol) CsF zugesetzt.

Ausbeute : 11,4 g 2.2.3.3.-Tetrafluor-1.4-dioxan ≙ 14 % d. Th.

Beispiel 6

In einen trockenen 500 ml Kolben mit KPG-Rührer, Rückflußkühler, Thermometer und Tropftrichter gibt man 200 ml Tetraglyme und 95 g (0,625 Mol) Caesiumfluorid. Dann tropft man unter Eiskühlung 57 g (0,294 Mol) Hexafluordiacetyl zu. Der Tropftrichter wird danach entfernt und 80 g (0,37 Mol) Glykoldimesylat zugegeben. Anschließend wird der Ansatz 14 Stunden bei 100-105 °C gerührt. Mit einer Wasserdampfdestillation werden 45 g organisches Produkt aus dem Reaktionsgemisch abgetrennt. Die Destillation liefert 35 g (46 % Ausbeute) an 2.3-Bis-trifluormethyl-2.3-difluor-1.4-dioxan.

$C_6H_4F_8O_2$ Ber. : C 27,71 H 1,55 F 58,44
MG 260 Gef. : C 27,75 H 1,50 F 57,95
Kp 100 mm Hg (13332 Pa) 75-90 °C (Diastereomerengemisch)

**Ansprüche**

1. Verfahren zur Herstellung von 2.3-Perfluor- bzw. -trifluormethyl-1,4-dioxanen der allgemeinen Formel I

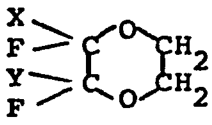

(I)

worin X und Y unabhängig voneinander F oder $CF_3$ bedeuten, dadurch gekennzeichnet, daß man Carbonylverbindungen der allgemeinen Formel

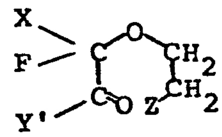 (II)

worin

X die gleiche Bedeutung wie in Formel I besitzt,

Y' die gleiche Bedeutung wie Y in Formel I hat (F, $CF_3$), und zusätzlich noch Cl sein kann, und

Z = Cl, Mesylat [= $OSO_2CH_3$]- oder Tosylat [= $OSO_2C_6H_4—CH_3$(p)]-rest

mit Alkalifluoriden und/oder mit Ammoniumfluorid in einer Menge von mindestens etwa 1 Mol Fluorid/Mol Carbonylverbindung II, wenn Y' = F oder $CF_3$, oder von mindestens etwa 2 Mol Fluorid/Mol Carbonylverbindung II, wenn Y' = Cl, umsetzt.

2. Verfahren nach Anspruch 1, dadurch gekennzeichnet, daß man die Carbonylverbindungen der Formeln II mit Z = Mesylatrest oder Tosylatrest in situ herstellt durch Umsetzung von Fluorcarbonylverbindungen V

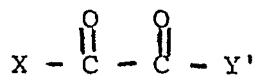 (V)

mit Ethanderivaten VI

$$Z—CH_2—CH_2—Z \quad (VI),$$

wobei in den Formeln X und Y' die gleiche Bedeutung wie in Formel II besitzen und Z = Mesylatrest oder Tosylatrest, und Alkalifluoriden und/oder Ammoniumfluorid.

3. Verfahren nach den Ansprüchen 1 bis 2, dadurch gekennzeichnet, daß man die Umsetzung der Carbonylverbindungen II mit KF und/oder CsF durchführt.

4. Verfahren nach den Ansprüchen 1 bis 3, dadurch gekennzeichnet, daß man die Alkalifluoride und/oder das Ammoniumfluorid in einem 5 bis 50 %igen Überschuß verwendet.

5. Verfahren nach den Ansprüchen 1 bis 4, dadurch gekennzeichnet, daß man die Umsetzung in aprotisch-polaren Lösungsmitteln durchführt.

6. Verfahren nach den Ansprüchen 1 bis 5, dadurch gekennzeichnet, daß man die Umsetzung bei Temperaturen zwischen 20 und 200 °C, vorzugsweise zwischen 50 und 160 °C, insbesondere zwischen 60 und 130 °C, durchführt.

7. Verfahren nach den Ansprüchen 1 bis 6, dadurch gekennzeichnet, daß man die Umsetzung in einer Zeit zwischen 5 und 35 Stunden durchführt.

**Claims**

1. Process for the manufacture of 2.3-perfluoro- or -trifluoromethyl-, resp., -1.4-dioxanes of the general formula I

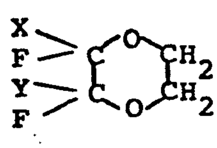 (I)

wherein X and Y independently from one another mean F or $CF_3$, characterized in reacting carbonyl compounds of the general formula

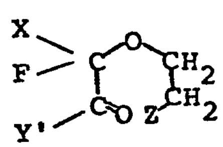 (II)

wherein

X has the same meaning as in formula I,

Y' has the same meaning as Y in formula I (F, $CF_3$) and additionally can be Cl, and

Z = Cl, mesylate [= $OSO_2CH_3$] or tosylate [= $OSO_2C_6H_4—CH_3$(p)]

with alkali metal fluorides and/or ammonium fluoride in an amount of at least about 1 mol of fluoride/mol of carbonyl compound II if Y' is F or $CF_3$, or of at least about 2 mols of fluoride/mol of carbonyl compound II, if Y' = Cl.

2. Process according to claim 1, characterized in manufacturing the carbonyl compounds of the formula II with Z being mesylate or tosylate, in situ by reaction of fluorocarbonyl compounds V

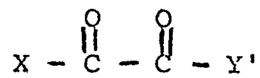

(V)

with ethane derivatives VI

$$Z—CH_2—CH_2—Z \quad (VI),$$

with X and Y' in the formula V having the same meaning as in formula II and with Z being mesylate or tosylate and with alkali metal fluorides and/or ammonium fluoride.

3. Process according to claims 1 and 2, characterized in carrying out the reaction of the carbonyl compounds II with KF and/or CsF.

4. Process according to claims 1 to 3, characterized in using the alkali metal fluorides and/or the ammonium fluoride in an excess of 5 to 50 %.

5. Process according to claims 1 to 4, characterized in performing the reaction in aprotonic-polar solvents.

6. Process according to claims 1 to 5, characterized in carrying out the reaction at temperatures between 20 and 200 °C, preferably between 50 and 160 °C, especially between 60 and 130 °C.

7. Process according to claims 1 to 6, characterized in carrying out the reaction within a period between 5 and 35 hours.

**Revendications**

1. Procédé de préparation de perfluoro- ou trifluorométhyl-2,3 dioxannes-1,4 répondant à la formule générale I

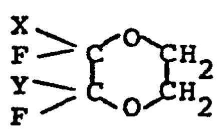

(I)

dans laquelle X et Y représentent chacun, indépendamment l'un de l'autre, F ou $CF_3$, procédé caractérisé en ce qu'on fait réagir des composés carbonyliques répondant à la formule générale II

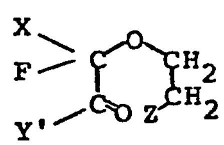

(II)

dans laquelle

X a la même signification que dans la formule I,

Y' a la même signification que Y dans la formule I (c'est-à-dire F ou $CF_3$) et peut en outre représenter Cl et

Z représente Cl ou un radical mésylate [$—OSO_2CH_3$] ou tosylate [$—OSO_2—C_6H_4—CH_3$ (p)],

avec des fluorures de métaux alcalins et/ou avec le fluorure d'ammonium, en une quantité d'au moins environ 1 mole du fluorure par mole du composé carbonylique (II) dans le cas où Y' représente F ou $CF_3$, et d'au moins environ 2 moles du fluorure par mole du composé carbonylique (II) dans le cas où Y' représente Cl.

2. Procédé selon la revendication 1, caractérisé en ce que les composés carbonyliques de formule II dans laquelle Z représente un radical mésylate ou tosylate sont préparés in situ par réaction de composés fluorocarbonyliques de formule V

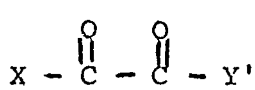

(V)

avec des dérivés de l'éthane de formule VI

$$Z-CH_2-CH_2-Z \qquad (VI)$$

formules dans lesquelles X et Y' ont les mêmes significations que dans la formule II, et Z représente un radical mésylate ou tosylate, et avec des fluorures de métaux alcalins et/ou du fluorure d'ammonium.

3. Procédé selon l'une des revendications 1 et 2, caractérisé en ce qu'on effectue la réaction des composés carbonyliques (II) avec KF et/ou CsF.

4. Procédé selon l'une quelconque des revendications 1 à 3, caractérisé en ce que les fluorures de métaux alcalins et/ou le fluorure d'ammonium sont utilisés en un excès de 5 à 50 %.

5. Procédé selon l'une quelconque des revendications 1 à 4, caractérisé en ce qu'on effectue la réaction dans des solvants polaires aprotiques.

6. Procédé selon l'une quelconque des revendications 1 à 5, caractérisé en ce qu'on effectue la réaction à des températures comprises entre 20 et 200 °C, de préférence entre 50 et 160 °C ou, mieux encore, entre 60 et 130 °C.

7. Procédé selon l'une quelconque des revendications 1 à 6, caractérisé en ce qu'on effectue la réaction en un temps compris entre 5 et 35 heures.